# DEMANDE DE BREVET EUROPEEN

(11) **EP 0 940 137 A1**
(43) Date de publication de la demande: **08.09.1999**
(21) Numéro de dépôt: 98403229.2
(22) Date de dépôt: 18.12.1998
(51) Int. Cl.: A61K 7/48, A61K 7/027, A61K 7/025

(54) **Composition de soin des lèvres contenant de l'acide acexamique, ses utilisations**

(30) Priorité: 13.01.1998 FR 9800247; 05.03.1998 FR 9802717
(71) Demandeur: L'OREAL, 75008 Paris (FR)
(72) Inventeur: Breton, Lionel, 78000 Versailles (FR); Arnaud, Pascal, 94240 l'Hay-les-Roses (FR)
(74) Mandataire: Lhoste, Catherine

(57) **Abrégé**

L'invention se rapporte à une composition notamment anhydre de soin des lèvres contenant comme actif de soin physiologiquement acceptable de l'acide acexamique solubilisé ou dispersé dans une phase grasse. Cette composition peut se présenter sous forme de stick notamment de rouge à lèvres ou de baume à lèvres pour protéger les lèvres en particulier contre les intempéries et éviter ainsi leur dessèchement et/ou la formation de gerçures et de crevasses.

## Description

La présente invention se rapporte à une composition de soin et/ou de traitement des lèvres des êtres humains, contenant de l'acide acexamique [appelé aussi l'acide acétamidocaproïque ou encore l'acide 6-(acétylamino) hexanoïque]. Cette composition est utilisable dans le domaine cosmétique ou dermatologique. Plus spécialement cette composition se présente sous forme d'une gel, crème ou stick anhydre homogène, éventuellement colorée, renfermant cet actif. En particulier, cette composition permet d'éviter le dessèchement, la formation de gerçure ou de crevasse ainsi que le traitement des gerçures et crevasses existantes. Elle assure, en outre, souplesse et douceur aux lèvres ainsi qu'une résorption des oedèmes et une suppression des douleurs labiales.

Pour protéger les lèvres contre le dessèchement, on utilise généralement des baumes à lèvres en stick, contenant des huiles traitantes, généralement d'origine végétale telles que les triglycérides (voir à cet effet le document JP-A-04 360 810). Malheureusement, même en quantité élevée, ces huiles ne permettent pas un traitement suffisant des lèvres, surtout lorsque ces dernières sont gonflées ou pourvues de crevasses ou de gerçures. En outre, plus la quantité de ces huiles augmentent plus, le toucher du baume est gras, conférant un aspect peu esthétique des lèvres ainsi que des propriétés cosmétiques d'application médiocres (gras, lourdeur). Il est donc souhaitable de (imiter cet aspect gras dans un produit de maquillage des lèvres. Par ailleurs, il est à ce jour difficile d'obtenir des produits pour lèvres, à propriétés traitantes, ne contenant pas ou peu d'huile d'origine végétale.

L'invention a justement pour objet une composition de soin et/ou de traitement des lèvres permettant de remédier à ces inconvénients. De façon surprenante, le demandeur a trouvé que l'utilisation d'acide acexamique dans une base de soin ou de maquillage des lèvres, de tout type, permettait de les traiter, d'empêcher leur dessèchement et la formation de gerçures et/ou de crevasses. La composition permet notamment un maquillage des lèvres et/ou une protection des lèvres, esthétique tout en traitant celles-ci, et ce quelque soit la phase grasse de ladite composition.

L'invention s'applique non seulement aux produits de soin et/ou de traitement des lèvres, mais aussi aux produits de maquillage des lèvres ayant des propriétés de soin et/ou de traitement.

De façon plus précise, l'invention a pour objet une composition de soin ou de maquillage des lèvres contenant de l'acide acexamique, comme actif de soin physiologiquement acceptable, et une phase grasse renfermant cet actif, ladite phase grasse contenant au moins une huile choisie parmi les huiles hydrocarbonées ayant au moins 4 atomes de carbone, les huiles siliconées, les huiles fluorées et leurs mélanges.

L'acide acexamique peut être utilisé notamment à raison de 0,05 à 10 % et mieux de 0,1 à 5 % du poids total de la composition. Il peut se présenter sous forme libre ou sous forme de sel et notamment sous forme de sel de zinc.

L'acide acexamique se présente en particulier sous forme de poudre, ce qui permet son incorporation dans la phase grasse, sous forme soluble ou dispersée, de façon aisée, ce qui n'est pas toujours le cas pour un actif sous forme liquide (notamment problème de stabilisation d'une émulsion et nécessité d'un appareillage sophistiqué pour faire certaines émulsions).

Cette composition peut être utilisée telle quelle ou bien être incorporée dans une composition plus complexe. Elle est notamment non collante au toucher, non grasse et douce à l'application, tout en traitant de manière satisfaisante les lèvres.

La composition de l'invention peut se présenter sous forme de pâte, de solide, de crème ou même de liquide. Elle peut être une émulsion huile-dans-eau ou eau-dans-huile, un gel anhydre, solide ou souple ou une phase huileuse liquide. De préférence, elle se présente sous forme de composition solide anhydre, et plus spécialement sous forme de stick.

De préférence la composition contient une certaine quantité d'huile hydroxylée en vue de solubiliser l'acide acexamique ou de le disperser correctement. Avantageusement, ces huiles présentent un indice de groupe hydroxyle ≥50 et < 750. Elles présentent, en outre, avantageusement au moins 4 atomes de carbone.

Comme huiles hydroxylées utilisables dans l'invention, on peut citer notamment :
- les esters et les éthers de synthèse comme le C₁₂-C₁₃ alkyl lactate, l'isostéaryl lactate, l'hydroxystéarate d'octyle, l'hydroxystéarate d'octyldodécyle, le di-isostéaryl malate, le mono-isostéarate de propylène glycol, le polyglycéryl 3- di-isostéarate, le PPG 10 butane diol ;
- les triglycérides liquides comme l'huile de ricin ;
- les alcools gras en C₁₂ à C₂₆ comme l'octyl dodécanol, le 2-hexyldécanol, le 2-undécylpentadécanol ou l'alcool oléique ;
- leurs mélanges.

Il est toutefois possible d'utiliser des huiles non hydroxylées associées ou non aux huiles hydroxylées ci-dessus. Ces huiles non hydroxylées peuvent être des huiles hydrocarbonées, des huiles siliconées et/ou des huiles fluorées. Comme huile non hydroxylée, hydrocarbonée, utilisable dans l'invention, on peut citer :
- les triglycérides liquides d'acides gras de 4 à 10 atomes de carbone, linéaires ou ramifiés, saturés ou insaturés, liquides à température ambiante, comme les triglycérides des acides heptanoïque ou octanoïque, les huiles de tournesol, de maïs, de germe de blé, de soja, de courge, de pépins de raisin ou de cassis, d'onagre, de millet, d'orge, de quinoa, d'olive, de seigle, de carthame, de bancoulier, de passiflore, de rosier muscat, de sésame, de noisette, d'abricot, de macadamia, d'amandes douces, d'avocat, de coton, de luzerne, de pavot, de potimarron, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba ou de beurre de karité ;
- les esters et les éthers de synthèse notamment d'acides gras comme les huiles de formule R₃COOR₄ dans laquelle R₃ représente le reste d'un acide gras supérieur en C₇ à C₂₉ et R₄ représente une chaîne hydrocarbonée en C₃ à C₃₀ comme l'huile de Purcellin, le myristate d'isopropyle, le palmitate d'éthyl-2-hexyle, le stéarate d'octyl-2-dodécyle, le stéarate d'octacosanyle, l'érucate d'octyl-2-dodécyle, l'isostéarate d'isostéaryle ; le citrate de triisocétyle, les heptanoates, octanoates, décanoates ou ricinoléates d'alcools gras ; les esters de polyol comme le dioctanoate de propylène glycol, le diheptanoate de néopentylglycol, le diisononanoate de diéthylèneglycol et les esters du pentaérythritol ;
- les hydrocarbures linéaires ou ramifiés d'origine synthétique ou minérale comme les huiles de paraffine volatiles ou non et leurs dérivés, la vaseline, les polydécènes, le polyisobutène hydrogéné tel que le parléam ou le perhydrosqualène ;
- leurs mélanges.

Les huiles siliconées utilisables dans l'invention sont notamment les polydiméthylsiloxanes (PDMS) volatiles ou non, linéaires ou cycliques, liquides ou pâteux à température ambiante, les polydiméthylsiloxanes comportant des groupements alkyle, alcoxy ou phényle, pendant ou en bout de chaîne siliconée, groupements ayant de 2 à 24 atomes de carbone comme les phényltriméthicones, les diphényl diméthicones, les phényl diméthicones, les polyméthylphényl siloxanes, les diphénylméthyldiméthyltrisiloxanes les phényltriméthylsiloxydiphényl siloxanes. Les huiles siliconées peuvent, en outre, être eventuellement fluorées.

Les huiles hydroxylées représentent notamment de 0,2 à 99,95 % du poids total de la composition, de préférence de 1 à 80 %. Les huiles non hydroxylées représentent de 0 à 99,95 % du poids de la composition et mieux de 0 à 50 %.

La composition de l'invention peut comprendre, en outre, tout ingrédient usuellement utilisé dans le domaine concerné, tel que l'eau, notamment en une quantité allant de 0 à 95 % du poids total de la composition, des colorants hydrosolubles ou liposolubles, des antioxydants, des huiles essentielles, des conservateurs, des parfums, des neutralisants, des polymères liposolubles notamment hydrocarbonés tels que les polyalkylènes ou le polylaurate de vinyle, des gélifants de phase aqueuse, des gélifiants de phase grasse liquide, des cires, des gommes, des tensioactifs, des actifs cosmétiques ou dermatologiques additionnels comme par exemple des émollients, des hydratants (glycérine par exemple), des vitamines, de la lanoline liquide, des acides gras essentiels, des filtres solaires lipophiles ou hydrophiles, et leurs mélanges. La composition selon l'invention peut contenir également des vésicules lipidiques de type ionique et/ou non ionique. Ces ingrédients, autres que l'eau) peuvent être présents dans la composition à raison de 0 à 20% du poids total de la composition.

Bien entendu l'homme du métier veillera à choisir les éventuels ingrédients complémentaires et/ou leur quantité de telle manière que les propriétés avantageuses de la composition selon l'invention ne soient pas, ou substantiellement pas, altérées par l'adjonction envisagée.

La composition selon l'invention se présente avantageusement sous forme épaisse anhydre. Aussi, l'invention se rapporte plus spécialement à une composition anhydre de maquillage ou de soin des lèvres, épaissie contenant au moins un agent épaississant choisi parmi les gélifiants de phase grasse, les cires, les charges et leurs mélanges. Les cires peuvent être hydrocarbonées, fluorées et/ou siliconées et être d'origine végétale, minérale, animale et/ou synthétique. En particulier, les cires présentent une température de fusion supérieure à 25 °C et mieux supérieure à 45 °C.

Comme gélifiant de phase grasse, on peut citer les argiles éventuellement modifiées comme les hectorites modifiées par un chlorure d'ammonium d'acide gras en C₁₀ à C₂₂, comme l'hectorite modifiée par du chlorure de di-stéaryl di-méthyl ammonium ; la silice ; les organopolysiloxanes élastomériques partiellement ou totalement réticulés, de structure tridimensionnelle, comme ceux commercialisés sous les noms KSG6, KSG16, KSG18 de Shin-Etsu, Trefil E-505C ou Trefil E-506C de Dow-Corning, Gransil SR-CYC, SR DMF10, SR-DC556, SR 5CYC gel, SR DMF 10 gel, SR DC 556 gel de Grant Industries, SF 1204 et JK 113 de General Electric ; les galactommananes comportant un à six et mieux de deux à quatre groupes hydroxyle par ose, substitués par une chaîne alkyle saturée ou non, comme la gomme de guar alkylée par des chaînes alkyle en C₁ à C₆ et mieux en C₁ à C₃ et plus particulièrement la guar éthylée ayant un degré de substitution de 2 à 3 telle que celle vendue par la société Aqualon sous le nom N-HANCE-AG ; les gommes notamment siliconées comme les PDMS ayant une viscosité > 500 000 centistokes. Ces gélifiants sont utilisés par exemple à des concentrations de 0,2 à 15 % du poids total de la composition.

Comme cire utilisable dans l'invention, on peut citer des cires hydrocarbonées, siliconées et/ou fluorées, comportant éventuellement des fonctions ester, hydroxyle, ou thiol. A titre d'exemple, on peut citer, la lanoline, la cire d'abeilles, la cire de Carnauba ou de Candellila, la paraffine, les cires de lignite ou microcristalline, la cérésine ou l'ozokérite ; les cires synthétiques comme les cires de polyéthylène, les cires de silicones comme les alkyl ou alkoxy-diméticone ayant de 16 à 45 atomes de carbone, les cires de Fischer-Tropsch, et leurs mélanges.

La nature et la quantité des cires sont fonction des propriétés mécaniques et de textures recherchées. A titre indicatif, la composition peut contenir de 0 à 50 % en poids de cires, par rapport au poids total de la composition et mieux de 5 à 30 %. Ces cires sont, en outre, des agents structurants de la composition.

La composition selon l'invention peut se présenter sous la forme d'une composition dermatologique ou de soin des lèvres, ou sous forme d'une composition de protection solaire des lèvres. Elle se présente alors sous forme non colorée, contenant éventuellement des actifs cosmétiques ou dermatologiques, autres que l'acide acexamique. Elle peut alors être utilisée comme base de soin pour les lèvres (baumes à lèvres, protégeant les lèvres du froid et/ou du soleil et/ou du vent).

La composition de l'invention peut également se présenter sous la forme d'un produit coloré de maquillage des lèvres, comme un rouge à lèvres ou un brillant à lèvres, présentant des propriétés de soin ou de traitement. Aussi, l'invention a encore pour objet une composition anhydre de maquillage des lèvres épaissie contenant de l'acide acexamique, comme actif de soin physiologiquement acceptable, et une phase grasse renfermant cet actif et un agent épaississant.

Bien entendu la composition de l'invention doit être cosmétiquement ou dermatologiquement acceptable, à savoir non toxique et susceptible d'être appliquée sur les lèvres d'êtres humains.

La composition de l'invention peut comprendre, avantageusement une phase particulaire, généralement présente à raison de 0 à 35 % du poids total de la composition, de préférence de 0,5 à 25 %, et qui peut comprendre des pigments et/ou des nacres et/ou des charges habituellement utilisés dans les compositions cosmétiques ou dermatologiques.

Par pigments, il faut comprendre des particules blanches ou colorées, minérales ou organiques, insolubles dans la phase grasse liquide, destinées à colorer et/ou opacifier la composition. Par charges, il faut comprendre des particules incolores ou blanches, minérales ou de synthèse, lamellaires ou non lamellaires. Par nacres, il faut comprendre des particules irisées, notamment produites par certains mollusques dans leur coquille ou bien synthétisées. Ces charges et nacres servent notamment à modifier la texture de la composition et font notamment partie des agents structurants susceptibles de conduire à une forme solide.

Les pigments peuvent être présents dans la composition à raison de 0,05 à 25 % du poids de la composition finale, et de préférence à raison de 2 à 15 %. Comme pigments minéraux utilisables dans l'invention, on peut citer les oxydes de titane, de zirconium ou de cérium ainsi que les oxydes de zinc, de fer ou de chrome et le bleu ferrique. Parmi les pigments organiques utilisables dans l'invention, on peut citer le noir de carbone, et les laques de baryum, strontium, calcium (DC Red N°7), aluminium.

Les nacres peuvent être présentes dans la composition à raison de 0 à 20 % du poids total de la composition, de préférence à un taux de l'ordre de 1 à 15 %. Parmi les nacres utilisables dans l'invention, on peut citer le mica recouvert d'oxyde de titane, d'oxyde de fer, de pigment naturel ou d'oxychlorure de bismuth tel que le mica titane coloré.

Les charges peuvent être présentes à raison de 0 à 35 % du poids total de la composition, de préférence 0,5 à 15 %. On peut notamment citer le talc, le mica, le kaolin, les poudres de Nylon (Orgasol notamment) et de polyéthylène, le Téflon, l'amidon, le nitrure de bore, des microsphères de copolymères telles que l'Expancel (Nobel Industrie), le polytrap (Dow Corning) et les microbilles de résine de silicone (Tospearl de Toshiba, par exemple).

La composition selon l'invention peut être fabriquée par les procédés connus, généralement utilisés dans le domaine cosmétique ou dermatologique. En particulier, elle peut être obtenue par chauffage des différents constituants à la température de fusion des cires les plus élevées, puis coulage du mélange fondu dans un moule (coupelle ou doigt de gant). Elle peut aussi être obtenue par extrusion comme décrit dans la demande EP-A-667 146.

En vue d'assurer un protection efficace contre les rayonnements U.V., il est avantageux d'adjoindre à la composition un ou plusieurs filtres lipophiles ou hydrophiles, organiques ou minéraux.

L'invention a encore pour objet un procédé cosmétique de soin ou de traitement des lèvres des êtres humains, comprenant l'application sur les lèvres, notamment desséchées, gercées ou crevassées, de la composition telle que définie ci-dessus.

L'invention a encore pour objet l'utilisation de l'acide acexamique dans une composition cosmétique ou pour la fabrication d'une composition dermatologique pour limiter, voire supprimer, le dessèchement et/ou les oedèmes des lèvres, les gerçures, les crevasses, et/ou protéger les lèvres des intempéries.

L'invention est illustrée plus en détail dans les exemples suivants. Les pourcentages sont donnés en poids.

### Exemple 1 : stick de soin des lèvres

| | |
|---|---|
| - Huile de ricin | 11,14 % |
| - Hydroxystéarate d'hydroxy octacosanyle | 3,74 % |
| - Stéarate octacosanyle | 7,54 % |
| - Parfum | 0,50 % |
| - Hectorite modifiée par chlorure de di-stéaryl di-méthyl ammonium | 0,66 % |
| - Acide acexamique | 0,30 % |
| - Polylaurate de vinyle | 8,90 % |
| - Cire de polyéthylène (Poids Moléculaire 500) | 3,67 % |
| - Mono-isostéarate de propylène glycol | qsp 100 % |

*Préparation* : On dissout préalablement l'acide acexamique dans l'huile de ricin à 100 °C. On ajoute ensuite le reste des constituants à l'exception du parfum et on fond l'ensemble à 100 °C. Après homogénéisation, on introduit le parfum puis on coule l'ensemble dans un moule approprié à l'obtention d'un stick.

On obtient un stick anhydre blanc, de texture agréable, qui glisse bien et s'applique bien sur les lèvres, ayant des propriétés traitantes des lèvres, et assurant notamment une protection contre le dessèchement et les gerçures.

Ce baume a été testé par 30 femmes, en application quotidienne pendant 1 semaine, ayant des lèvres desséchées et utilisatrices de ce type de produit, et sans changer leurs habitudes de maquillage des lèvres. Les résultats ci-après montre une nette amélioration de l'état des lèvres en fin de traitement.
- 69 % des femmes observent des lèvres plus lisses, 49 % de ces femmes des lèvres plus douces et 31 % de ces femmes les lèvres plus souples.
- 20 femmes sur les 30 trouvent un confort à l'application bon ou très bon, une bonne texture, une application aisée, quoique le baume soit ressenti comme légèrement gras.

### Exemple 2 : Baume pour les lèvres

| | |
|---|---|
| - Cire de Carnauba | 12,75 % |
| - Huile de ricin | qsp 100 % |
| - Acide acexamique | 0,25 % |
| - Cire de lanoline oxypropylènée (5 Oxydes de propylène) | 15,00 % |

Après fusion des cires de Carnauba et de lanoline à 100 °C, on introduit l'huile de ricin contenant l'acide acexamique, puis on mélange l'ensemble et coule le tout dans un moule approprié.

### Exemple 3 : Baume pour les lèvres

| | |
|---|---|
| - Cire de polyéthylène (P.M. 500) | 3,30 % |
| - Mono-isostéarate de propylène glycol | qsp 100 % |
| - Acide acexamique | 0,25 % |
| - Cire de lanoline oxypropylénée (5 Oxydes de propylène) | 15,00 % |
| - Stéarate d'octacosanyle | 7,60 % |
| - Hydroxystéarate d'hydroxy octacosanyle (cire) | 4,20 % |

Ce baume à lèvres est doux et glissant.

### Exemple 4 : Baume pour les lèvres

| | |
|---|---|
| - Cire de polyéthylène (P.M. 500) | 3,30 % |
| - Mono-isostéarate de propylène glycol | qsp 100 % |
| - Acide acexamique | 0,25 % |
| - Cire de lanoline oxypropylénée (5 Oxyde de propylène) | 12,00 % |
| - Stéarate d'octacosanyle | 7,60 % |
| - Hydroxystéarate d'hydroxy octacosanyle | 4,20 % |
| - Polybutène | 10,00 % |

Ce baume est moins glissant et légèrement moins gras que celui de l'exemple 3.

### Exemple 5 : Rouge à lèvres

| | |
|---|---|
| - Cire de polyéthylène (P.M. 500) | 3,30 % |
| - Mono-isostéarate de propylène glycol | qsp 100 % |
| - Acide acexamique | 0,25 % |
| - Cire de lanoline oxypropylénée (5 Oxydes de propylène) | 8,00 % |
| - Stéarate d'octacosanyle | 7,60 % |
| - Hydroxystéarate d'hydroxy octacosanyle | 4,20 % |
| - Polybutène | 10,00 % |
| - Pigments | 8,66 % |
| - Parfum | 0,50 % |

Les pigments sont un mélange d'oxyde de titane, d'oxyde de fer noir de laques organiques.

*Préparation* : On broie les pigments dans un mélange contenant une fraction de mono-isostéarate de propylène glycol, la cire de lanoline et le polybutène. On ajoute les autres cires et on fond l'ensemble à 100 °C. Après homogénéisation, on ajoute la solution d'acide acexamique obtenue à 100 °C dans le reste de mono-isostéarate de propylène glycol. On ajoute le parfum puis on coule dans un moule approprié à l'obtention d'un stick.

### Exemple 6 : Rouge à lèvres

| | |
|---|---|
| - Cire de polyéthylène (P.M. 500) | 3,35 % |
| - Mono-isostéarate de propylène glycol | qsp 100 % |
| - Acide acexamique | 0,30 % |
| - Polylaurate de vinyle | 8,00 % |
| - Stéarate d'octacosanyle | 6,84 % |
| - Hydroxystéarate d'hydroxy octacosanyle | 3,40 % |
| - Huile de ricin | 10,00 % |
| - Quarternium 18 hectorite (bentone) | 1,20 % |
| - Pigments | 8,66 % |
| - Parfum | 0,50 % |

Ce rouge à lèvres est préparé comme celui de l'exemple 5.

### Exemple7 : Rouge à lèvres

| | |
|---|---|
| - Cire de polyéthylène (P.M. 500) | 12,00 % |
| - Lanoline liquide | 15,00 % |
| - Phényltriméthicone (DC556 de Dow Corning) | 63,34 % |
| - Pigments | 8,66 % |
| - Acide acexamique | 1,00 % |

*Preparation* : On broie les pigments et l'acide acexamique dans un mélange contenant de la phényltriméthicone et la lanoline liquide. On fait fondre le reste des constituants à 100°C puis on les ajoute au mélange. Après homogénéisation, on coule l'ensemble dans un moule approprié. Dans ce rouge à lèvres, l'acide acexamique est dispersé.

## Revendications

1. Composition de soin ou de maquillage des lèvres contenant de l'acide acexamique, comme actif de soin physiologiquement acceptable, et une phase grasse renfermant cet actif, ladite phase grasse contenant au moins une huile choisie parmi les huiles hydrocarbonées contenant au moins 4 atomes de carbone, les huiles siliconées, les huiles fluorées et leurs mélanges.

2. Composition selon la revendication 1, caractérisée en ce que l'acide acexamique est utilisé à raison de 0,05 à 10 % et mieux de 0,1 à 5 % du poids total de la composition.

3. Composition selon la revendication 1 ou 2, caractérisée en ce que l'acide acexamique se présente sous forme libre ou sous forme de sel.

4. Composition selon l'une des revendications précédentes, caractérisée en ce que la phase grasse contient au moins une huile hydroxylée.

5. Composition selon l'une des revendications précédentes, caractérisée en ce que la phase grasse contient de 0,2 à 99,95 % du poids total de la composition d'huile hydroxylée.

6. Composition selon l'une des revendications 4 ou 5, caractérisée en ce que l'huile hydroxylée est choisie parmi :
- les triglycérides liquides ;
- les esters et les éthers de synthèse ;
- les alcools gras en C₁₂ à C₂₆ ;
- leurs mélanges.

7. Composition selon l'une des revendications 4 à 6, caractérisée en ce que l'huile hydroxylée est choisie parmi :
- l'huile de ricin ,
- l'isostéaryl lactate, l'hydroxystéarate d'octyle, l'hydroxystéarate d'octyldodécyle, le di-isostéaryl malate, le mono-isostéarate de propylène glycol, le polyglycéryl 3- di-isostéarate, le PPG 10 butane diol;
- l'octyl dodécanol, le 2-hexyldécanol, le 2-undécylpentadécanol, ou l'alcool oléique ;
- leurs mélanges.

8. Composition selon l'une des revendications précédentes, caractérisée en ce qu'elle contient au moins une huile non hydroxylée choisie parmi :
- les triglycérides liquides d'acides gras de 4 à 10 atomes de carbone, linéaires ou ramifiés, saturés ou insaturés, liquides à température ambiante ;
- les esters et les éthers de synthèse notamment d'acides gras comme les huiles de formule R₃COOR₄ dans laquelle R₃ représente le reste d'un acide gras supérieur en C₇ à C₂₉ et R₄ représente une chaîne hydrocarbonée en C₃ à C₃₀ ;
- les hydrocarbures linéaires ou ramifiés d'origine synthétique ou minérale
- les polydiméthylsiloxanes (PDMS) volatiles ou non, linéaires ou cycliques, liquides ou pateux à température ambiante, les polydiméthylsiloxanes comportant des groupements alkyle, alcoxy ou phényle, pendant ou en bout de chaîne siliconée, groupements ayant de 2 à 24 atomes de carbone ;
- leurs mélanges.

9. Composition selon l'une des revendications précédentes, caractérisée en ce qu'elle contient au moins un actif cosmétique et/ou dermatologique, différent de l'acide acexamique.

10. Composition selon l'une des revendications précédentes, caractérisée en ce qu'elle contient au moins un actif cosmétique et/ou dermatologique choisi parmi les émollients, les hydratants, les vitamines, la lanoline, les acides gras essentiels, les filtres solaires, les oxydes de titane ou de fer.

11. Composition selon l'une des revendications précédentes, caractérisée en ce qu'elle contient au moins une charge particulaire.

12. Composition selon l'une des revendications précédentes, caractérisée en ce qu'elle se présente sous forme d'une émulsion huile-dans-eau ou eau-dans-huile, d'une composition anhydre solide ou souple.

13. Composition selon l'une des revendications précédentes, caractérisée en ce qu'elle contient, en outre, au moins un ingrédient choisi parmi l'eau, les colorants hydrosolubles ou liposolubles, les antioxydants, les huiles essentielles, les conservateurs, les parfums, les gélifiants de phase aqueuse, les neutralisants, les polymères liposolubles, les gélifiants de phase grasse liquide, les cires, les gommes, les tensioactifs et leurs mélanges.

14. Composition selon l'une des revendications précédentes, caractérisée en ce qu'elle constitue un baume à lèvres ou un rouge à lèvres ou un brillant à lèvres, ayant des propriétés traitantes.

15. Composition selon l'une des revendications précédentes, caractérisée en ce qu'elle se présente sous forme de stick.

16. Composition anhydre de maquillage des lèvres, épaissie contenant de l'acide acexamique, comme actif de soin physiologiquement acceptable, et une phase grasse renfermant cet actif et un agent épaississant.

17. Composition selon la revendication 16, caractérisé en ce qu'elle contient, en outre, au moins une huile hydroxylée, une huile siliconée et/ou une charge particulaire.

18. Procédé cosmètique de soin ou de traitement des lèvres des êtres humains, comprenant l'application sur les lèvres de la composition cosmétique selon l'une quelconque des revendications précédentes.

19. Utilisation de l'acide acexamique dans une composition cosmétique ou pour la fabrication d'une composition dermatologique pour limiter, voire supprimer, le dessèchement et/ou les oedèmes des lèvres, les gerçures, les crevasses, et/ou protéger les lèvres des intempéries.
